# EUROPEAN PATENT SPECIFICATION

(11) **EP 1 678 158 B1**
(45) Date of publication and mention of the grant of the patent: **26.03.2008**
(21) Application number: 04817268.8
(22) Date of filing: 25.10.2004
(51) Int. Cl.: C07D 317/26

(54) **PROCESS FOR THE PREPARATION OF GLYCERALDEHYDE ACETONIDE**
VERFAHREN ZUR HERSTELLUNG VON GLYCERALDEHYDACETONID
PROCEDE DE PREPARATION DE GLYCERALDEHYDE ACETONIDE

(30) Priority: 28.10.2003 EP 03078392
(43) Date of publication of application: 12.07.2006
(73) Proprietor: DSM IP Assets B.V., 6411 TE Heerlen (NL)
(72) Inventor: QUAEDFLIEG, Peter, Jan, Leonard, Mario, NL-5583 ZG Waalre (NL); ALSTERS, Paulus, Lambertus, NL-6221 KZ Maastricht (NL); POJARLIEV, Peter, A-1120 Wien (AT); JARY, Walther, Gunther, A-4853 Steinbach am Attersee (AT)
(74) Representative: Breepoel, Peter M.
(86) International application number: PCT/EP2004/012064
(87) International publication number: WO 2005/040149

(56) References cited:
- EP-A- 0 775 684
- L. ERMOLENKO, ET AL.: "An expedient one-step preparation of (S)-2,3-O-isopropylideneglyceraldehyde" SYNLETT, no. 10, 28 September 2001 (2001-09-28), pages 1565-1566, XP002274843 THIEME VERLAG, STUTTGART, DE ISSN: 0936-5214 cited in the application
- K. OKADA, ET AL.: "Asymmetric synthesis of key synthetic intermediates of Aspidosperma and Hunteria type indole alkaloids" HETEROCYCLES, vol. 43, no. 8, 1 August 1996 (1996-08-01), pages 1735-1750, XP002274844 ELSEVIER SCIENCE PUBLISHERS, AMSTERDAM, NL ISSN: 0385-5414
- P.L. ANIELLI, ET AL.: "Fast and selection of primary alcohols to aldehydes or to carboxylic acids and of secondary alcohols to ketones mediated by oxoammonium salts under two-phase conditions" JOURNAL OF ORGANIC CHEMISTRY, vol. 52, no. 12, 12 June 1987 (1987-06-12), pages 2559-2562, XP002397801 AMERICAN CHEMICAL SOCIETY, WASHINGTON, DC, US

## Description

The invention relates to a process for the preparation of glyceraldehyde acetonide by oxidation of the corresponding 2,2-dimethyl-1,3-dioxolane-4-methanol by an oxidizing agent.

Such a process is known from Ermolenko et al. 2001, Synlett, 1565-1566 "An expedient one-step preparation of (S)-2,3-O-isopropylidene-glyceraldehyde". Ermolenko *et al*. disclose that (S)-glyceraldehyde acetonide can be prepared by oxidizing (R)-2,2-dimethyl-1,3-dioxolane-4-methanol with pyridinium chlorochromate (PCC) or pyridinium dichromate (PDC) in dichloromethane. A major disadvantage of this process is that very low yields (of 30%) are obtained due to severe by-product formation.

It is therefore the object of the invention to provide a process for the preparation of glyceraldehyde acetonide by oxidation of 2,2-dimethyl-1,3-dioxolane-4-methanol, wherein higher yields of glyceraldehyde acetonide are obtained.

This object is surprisingly achieved by oxidizing 2,2-dimethyl-1,3-dioxolane-4-methanol by an organic N-chloro compound in the presence of an inert base and TEMPO or a TEMPO-derivative of formula 1, 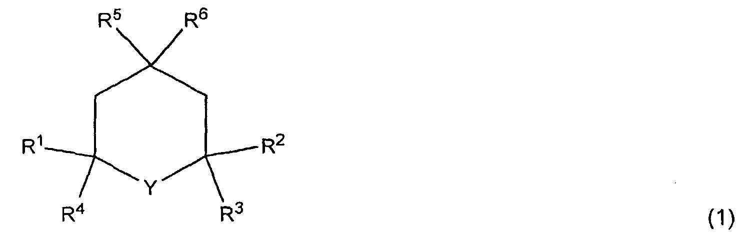 wherein R¹, R², R³ and R⁴ each independently stand for an alkyl group with 1 to 6 C-atoms and wherein R⁵ and R⁶ either both stand for H or an alkoxy group with 1 to 6 C-atoms or one stands for H and the other stands for an alkoxy group with 1 to 6 C-atoms, an alkylcarbonyloxy group with 1 to 6 C-atoms, an arylcarbonyloxy group with the carbonyloxy group having 1 to 6 C-atoms or an alkylcarbonylamino group with 1 to 6 C-atoms; or wherein R⁵ and R⁶ together stand for ketal groups of formula a-c 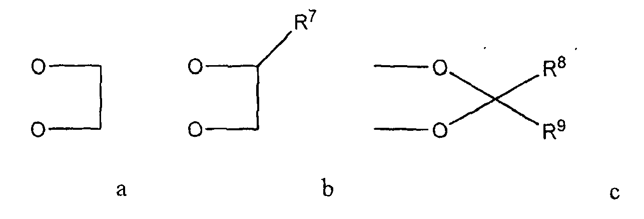 wherein R⁷ stands for an alkyl group with 1 to 6 C-atoms and R⁸ and R⁹ each independently stand for H or an alkyl group with 1 to 6 C-atoms and wherein Y stands for a group of general formula d-f 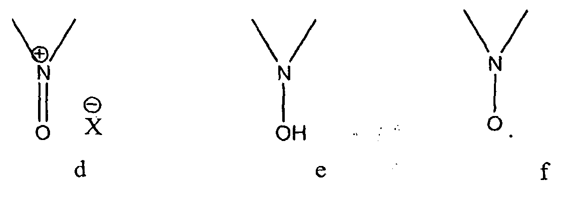 wherein X⁻ stands for an anion.

With this process a higher yield is achieved and substantially less byproducts are formed. Additionally, the process of the invention does not require the environmentally unfriendly oxidizing agents PCC or PDC.

The fact that primary and secondary alcohols can be oxidized to the corresponding aldehydes by an organic N-chloro compound in the presence of TEMPO or a TEMPO-derivative and a base is known from EP-B-0 775 684 and is shown for a specific set of alcohols. However, it is surprising that specifically this oxidation method works so well in the oxidation of 2,2-dimethyl-1,3-dioxolane-4-methanol to glyceraldehyde acetonide. This is surprising, because many other methods to oxidize alcohols are known and none of them has been found to be suitable for the oxidation of 2,2-dimethyl-1,3-dioxolane-4-methanol to glyceraldehyde acetonide in reasonable yields (see Table A).

Preferably, the process of the invention is performed in the presence of TEMPO (2,2,6,6-tetramethylpiperidinyloxy radical).

In one embodiment of the invention, enantiomerically enriched glyceraldehyde acetonide is prepared from the corresponding enantiomerically enriched 2,2-dimethyl-1,3-dioxolane-4-methanol. The enantiomerically enriched glyceraldehyde acetonide can either be (S)-glyceraldehyde acetonide or (R)-glyceraldehyde acetonide. Preferably, the glyceraldehyde acetonide has an enantiomeric excess (ee) > 80%, more preferably > 90%, in particular >95%, more in particular >98%, most in particular > 99%. Preferably, 2,2-dimethyl-1,3-dioxolane-4-methanol has an enantiomerica excess (ee) > 80%, more preferably > 90%, in particular >95%, more in particular >98%, most in particular > 99%. Surprisingly, with the process of the invention, the ee of the starting product, the corresponding 2,2-dimethyl-1,3-dioxolane-4-methanol is almost equal to the ee of the glyceraldehyde acetonide prepared. This implies that hardly any racemisation occurs during the process of the invention.

In the scope of the invention, with an inert base is meant a base which does not react (apart from usual acid-base reactions) with the organic N-chloro compound or one of its degradation products, TEMPO or a TEMPO-derivative of formula 1, wherein R¹, R², R³, R⁴, R⁵ and R⁶ are as defined above, nor with glyceraldehyde acetonide or 2,2-dimethyl-1,3-dioxolane-4-methanol. The inert base is used in the process of the invention to neutralize the HCl that is formed in the reaction. Preferably a base, which has a conjugated acid with a pKₐ > 2, is used. Examples of inert bases include sodium acetate and sodium bicarbonate.

Preferably an amount of base is used that is at least 0.8 molar equivalent based on the theoretically maximal molar amount of HCl that can be formed in the reaction, more preferably at least 0.9 molar equivalent, most preferably at least 1 molar equivalent.

The temperature of the process of the invention is in principle not critical. Preferably a temperature > -20°C, more preferably > 0°C, even more preferably > 15°C is used. The temperature is preferably < 100°C, more preferably < 80°C. In practice, it is preferred to use a temperature between -20 and 100°C, more preferably between 15 and 80°C.

The order of addition of the organic N-chloro compound, TEMPO or a TEMPO-derivative of formula 1, wherein R¹-R⁶ are as defined above, is in principle not critical. Preferably, TEMPO or a TEMPO-derivative of formula 1, wherein R¹-R⁶ are as defined above, is added to a mixture of 2,2-dimethyl-1,3-dioxolane-4-methanol, the organic N-chloro compound and the inert base in a solvent.

Suitable organic N-chloro compounds include N-chloro-4-toluenesulphonamide sodium salt, N-chloro-benzensulphonamide sodium salt, trichloroisocyanuric acid and dichlorodimethylhydantoin. Preferably, the organic N-chloro compound is trichloroisocyanuric acid or dichlorodimethylhydantoin.

The amount of organic N-chloro compound is in principle not critical. It is preferred to use an amount of organic N-chloro compound such that there is at least 0.5 molar equivalent active chlorine based on the amount of 2,2-dimethyl-1,3-dioxolane-4-methanol, more preferably at least 1 molar equivalent active chlorine and most preferably at least 1.1 molar equivalent active chlorine. The maximal amount of organic N-chloro compound is in principle not critical. For economical reasons, the amount of active chlorine is preferably less than 5 molar equivalent based on the amount of 2,2-dimethyl-1,3-dioxolane-4-methanol.

The amount of TEMPO or a TEMPO-derivative of formula 1, wherein R¹-R⁶ are as defined above, used in the process of the invention, is in principle not critical. However, for economical reasons, preferably < 20 mole%, more preferably < 5 mole%, in particular < 1 mole% TEMPO or a TEMPO-derivative (based on the amount of 2,2-dimethyl-1,3-dioxolane-4-methanol) is used. Preferably, the amount of TEMPO or a TEMPO-derivative (based on the amount of 2,2-dimethyl-1,3-dioxolane-4-methanol) is > 0.01 mole%, more preferably > 0.02 mole%, even more preferably >0.05 mole%, most preferably > 0.1 mole%. In practice, it is preferred to use an amount of TEMPO or a TEMPO-derivative of between 0.1 and 1 mole% based on the amount of 2,2-dimethyl-1,3-dioxolane-4-methanol.

Preferably the process of the invention is carried out in the presence of a solvent.

Suitable solvents include ketones, for example acetone, 2-butanone, methyl isobutyl ketone; esters, for example ethyl acetate or methyl acetate; halogenated hydrocarbons, for example dichloromethane; ethers, for example methyl-*t-*butylether or diethylether; aromatic solvents, for example toluene; nitriles, for example acetonitrile; amides, for example N,N-dimethylformamide; lactams, for example N-methyl pyrrolidinone; sulfoxides, for example dimethylsulfoxide. The preference for a solvent depends amongst others on the solubility of the chosen base and can easily be determined by the person skilled in the art.

Glyceraldehyde acetonide, in particular (S)-glyceraldehyde acetonide, is a useful intermediate in the synthesis of, for instance drugs, in particular anti-viral drugs, agrochemicals and the like. WO03/022853 describes for instance a process for the preparation of the following compounds (in particular the preparation of several compounds in enantiomerically enriched form): (2-(2,2-dimethyl-[1,3]dioxolan-4-ylmethylene)-malonic acid diethyl ester; 2-[1-(2,2-dimethyl-[1,3]dioxolan-4-yl)-2-nitroethyl]-malonic acid dimethyl ester; 4-methoxy-2-oxo-hexahydro-furo[3,4-*b*]furan-3-carboxylic acid methyl ester; 2-(4-hydroxy-2-methoxy-tetrahydro-furan-3-yl)-malonic acid dimethyl ester; 4-methoxy-tetrahydro-furo[3,4-*b*]furan-2-one, 4-hydroxy-2-methoxy-tetrahydro-furan-3-yl) acetic acid methyl ester; hexahydro-furo[2,3-*b*]furan-3-ol) starting from (*S*)-glyceraldehyde acetonide. These compounds can be used, in particular in enantiomerically enriched form, in the preparation of anti-viral drugs, in particular anti-HIV drugs, more in particular HIV protease inhibitors. These compounds will be indicated below using the reference numbers as used in WO03/022853. The compounds are of particular interest in preparing HIV protease inhibitors as disclosed in WO 95/24385, WO99/65870, WO 00/47551, WO 00/76961 and US 6,127,372, WO 01/25240, EP 0 715 618 and WO 99/67417, and in particular in preparing the following HIV protease inhibitors: [(1S,2R)-2-hydroxy-3-[[(4-methoxyphenyl)sulfonyl](2-methylpropyl)amino]-1-(phenylmethyl)propyl]-carbamic acid (3R,3aS, 6aR)-hexahydrofuro[2,3-b]furan-3-yl ester (HIV protease inhibitor 1); [(1S,2R)-3-[[(4-aminophenyl)sulfonyl](2-methylpropyl)amino]-2-hydroxy-1-(phenylmethyl)propyl]-carbamic acid (3R,3aS,6aR)-hexahydrofuro[2,3-b]furan-3-yl ester (HIV protease inhibitor 2); [(1S,2R)-3-[(1,3-benzodioxol-5-ylsulfonyl)(2-methylpropyl)amino]-2-hydroxy-1-(phenylmethyl)propyl]-carbamic acid (3R,3aS,6aR)-hexahydrofuro[2,3-b]furan-3-yl ester (HIVprotease inhibitor 3), or any pharmaceutically acceptable addition salt thereof.

According to WO03/022853, 2-(2,2-dimethyl-[1,3]dioxolan-4-ylmethylene)-malonic acid diethyl ester (compound III.2) can be prepared from glyceraldehyde acetonide using dimethylmalonate. 2-[1-(2,2-dimethyl-[1,3]dioxolan-4-yl)-2-nitroethyl]-malonic acid dimethyl ester (compound III.3) can be prepared by reaction of 2-(2,2-dimethyl-[1,3]dioxolan-4-ylmethylene)-malonic acid diethyl ester (compound III.2) with nitromethane in the presence of a catalytic amount of 1,8-diazabicyclo[5.4.0]undec-7-ene (DBU). 4-Methoxy-2-oxo-hexahydro-furo[3,4-*b*]furan-3-carboxylic acid methyl ester (compound III.4) and 2-(4-hydroxy-2-methoxy-tetrahydrofuran-3-yl)-malonic acid dimethyl ester (compound III.4') can be prepared from 2-[1-(2,2-dimethyl-[1,3]dioxolan-4-yl)-2-nitroethyl]-malonic acid dimethyl ester (compound III.3) by using first a base and subsequently an acid. The compounds 4-methoxy-tetrahydro-furo[3,4-*b*]furan-2-one (compound III.5) and 4-hydroxy-2-methoxy-tetrahydro-furan-3-yl) acetic acid methyl ester (compound III.5') may be prepared by decarboxylation of the compounds 4-methoxy-2-oxo-hexahydro-furo[3,4-*b*]furan-3-carboxylic acid methyl ester (compound III.4) and 2-(4-hydroxy-2-methoxy-tetrahydrofuran-3-yl)-malonic acid dimethyl ester (compound III.4'). Hexahydro-furo[2,3-*b*]furan-3-ol (compound 7.1) can be prepared by reduction of 4-methoxy-tetrahydro-furo[3,4-*b*]furan-2-one (compound III.5), which results in the intermediate compound: 4-(2-hydroxy-ethyl)-5-methoxy-tetrahydro-furan-3-ol (compound of formula (6)), which can then be cyclisized to form hexahydro-furo[2,3-*b*]furan-3-ol (compound 7.1.).

The process of the invention will now be elucidated by way of the following examples without however being limited thereto.

### Example 1. Screening of oxidants

### Materials and methods

(R,S)-Solketal (= (R,S)-2,2-dimethyl-1,3-dioxolane-4-methanol, 97 wt%) is used as received from Acros. 4-Methylmorpholine-*N*-oxide (monohydrate), pyridine-*N*-oxide (as applied in Expreiments E - H), RuCl₃, ZrOCl₂, TEMPO (= 2,2,6,6-tetramethylpiperidinyloxy radical); TCCA (= trichloroisocyanuric acid, 1 mole corresponds with 3 moles active chlorine) and NaOCl (aqueous solution, containing 12 wt% active chlorine) are used as received from Aldrich. Acetonitrile of p.a. quality (Merck) is used without further purification. H₂O₂ (50 wt% aqueous solution) is used as received from Degussa. Pyridine-N-oxide (as used in experiments I - L) is prepared in *situ* by treating a solution of pyridine (p.a. quality, Merck) in CHCl₃ (p.a. quality, Merck) at 0 °C with H₂O₂ (10 molar equivalent based on pyridine, 50 wt% aqueous solution, Degussa), stirring the reaction mixture overnight, separating the organic phase, washing it once with water and evaporating the solvent to give a residue which is used in the oxidation reaction without further purification.

For the GC analysis we used an Agilent 6890 GC combined with a DB-5 column with a length of 10 m, an internal diameter of 0.10 mm and a film thickness of 0.10 µm and using a split-flow inlet system (helium as carrier gas, column flow 0.5 mL/min and split 100:1). The injection volume was 1.0 µL. For detection an FID was used at 300 °C. The injection temperature was 250 °C and the column temperature programme consisted of 0.5 min at 100 °C followed by an increase to 280 °C with 40 °C/min.

### General procedure

0.50 g (3.79 mmole) (R,S)-Solketal is dissolved in 5 mL acetonitrile and subsequently 0.38 mmole (= 0.1 molar equivalent based on (R,S)-Solketal) of the catalyst (as shown in Table A) is added. To this solution was added (at room temperature and during 30 min.) a solution of the oxidant (1 or 2 molar equivalent based on (R,S)-Solketal, as shown in Table A). In case of 4-methylmorpholine-N-oxide, pyridine-*N*-oxide and TCCA the oxidant is added as a solution in 2 mL acetonitrile, in case of Pyr-NO, NaOCl and H₂O₂ the oxidant is added as an aqueous solution. The reaction mixture is stirred at room temperature and the (R,S)-Solketal conversion to (R,S)-Solketalaldehyde is monitored by GC. The maximal conversion measured is displayed in Table A.

### Results

**Table A. The conversion is defined as area% (R,S)-Solketalaldehyde divided by [area% (R,S)-Solketal + area% (R,S)-Solketalaldehyde] from the GC chromatogram.**

| Experiment | Oxidant | Molar equivalent oxidant* | Catalyst | Conversion (%) |
|---|---|---|---|---|
| A | 4-methylmorpholine-N-oxide | 1 | RuCl₃ | 7 |
| B | 4-methylmorpholine-N-oxide | 2 | RuCl₃ | 11 |
| C | 4-methylmorpholine-*N*-oxide | 1 | TEMPO | 2 |
| D | 4-methylmorpholine-*N*-oxide | 2 | TEMPO | 3 |
| E | Pyridine-*N*-oxide | 1 | ZrOCl₂ | 0 |
| F | Pyridine-*N*-oxide | 2 | ZrOCl₂ | < 1 |
| G | Pyridine-*N*-oxide | 1 | TEMPO | 0 |
| H | Pyridine-*N*-oxide | 2 | TEMPO | 0 |
| I | Pyridine-*N*-oxide** | 1 | RuCl₃ | < 1 |
| J | Pyridine-*N*-oxide** | 2 | RuCl₃ | < 1 |
| K | Pyridine-*N*-oxide** | 1 | ZrOCl₂ | 0 |
| L | Pyridine-*N*-oxide** | 2 | ZrOCl₂ | 0 |
| M | NaOCl | 1 | RuCl₃ | 0 |
| N | NaOCl | 2 | RuCl₃ | < 1 |
| O | NaOCl | 1 | ZrOCl₂ | 12 |
| P | NaOCl | 2 | ZrOCl₂ | 11 |
| Q | NaOCl | 1 | TEMPO | < 1 |
| R | NaOCl | 2 | TEMPO | < 1 |
| S | H₂O₂ | 1 | ZrOCl₂ | < 1 |
| T | H₂O₂ | 2 | ZrOCl₂ | 12 |
| U | TCCA | 0.4 | TEMPO | 44 |

| | | | | |
|---|---|---|---|---|
| * based on (R,S)-Solketal ** prepared *in situ* (see materials and methods) | | | | |

In conclusion, the above screening of oxidants shows that the combination of TCCA (an organic N-chloro compound) with TEMPO (or a TEMPO-derivative) used in the process of the invention (example U) is the only combination of oxidizing agent with catalyst that leads to an acceptable conversion of 2,2-dimethyl-1,3-dioxolane-4-methanol to glyceraldehyde acetonide.

### Example 2 Influence of parameters

### Materials and methods

(R)-Solketal (= (R)-2,2-dimethyl-1,3-dioxolane-4-methanol, 97 wt%, e.e. 99.6%), (R,S)-Solketal (= (R,S)-2,2-dimethyl-1,3-dioxolane-4-methanol, 97 wt%), TCCA (= trichloroisocyanuric acid, 99 wt%, 1 mole corresponds with 3 moles active chlorine), DCDMH (= dichlorodimethylhydantoin, 1 mole corresponds with 1.33 moles active chlorine), TEMPO (= 2,2,6,6-tetramethylpiperidinyloxy radical, 98 wt%), NaOAc (= sodium acetate, 99 wt%), NaHCO₃ (= sodium bicarbonate, 99 wt%), K₂CO₃ (= potassium carbonate, 99 wt%) and TEPA (= triethyl phosphonoacetate, 97 wt%) are used as received from Acros, as well as the solvents acetone, 2-butanone, ethyl acetate, acetonitrile and dichloromethane (all of p.a. quality).

For the GC analysis we used an Agilent 6890 GC (EPC) and an Agilent 7683 Automatic Liquid Sampler combined with a Betadex column (part number 24305, Supelco) with a length of 60 m, an internal diameter of 0.25 mm and a film thickness of 0.25 µm and using a split-flow inlet system with constant flow (Helium as carrier gas, column head pressure 26.4 kPa, column flow 1.4 mL/min and split flow 37.5 mL/min). For detection an FID was used at 250 °C. For the quantitative analytical determination of (R)- and (S)-glyceraldehyde acetonide the injection temperature was 150 °C and the column temperature programme consisted of 3 min at 60 °C, increase to 130 °C with 5 °C/min, another 1 min at 130 °C and increase to 230 °C with 25 °C/min. For the quantitative analytical determination of (R)- and (S)-ethyl-((4,5)-O-isopropylidene-(4,5)-dihydroxy)-2-(E,Z)-pentenoate the injection temperature was 250 °C and the column temperature programme consisted of 1 min at 80 °C, increase to 225 °C with 5 °C/min and another 10 min at 225 °C.

### Influence of the solvent

**Table 1: Influence of the solvent using 120 mole% NaOAc, 40 mole% TCCA and 0.25 mole% TEMPO based on Solketal.**

| Example | Solvent | Yield (%) of glyceraldehyde acetonide (based on Solketal) |
|---|---|---|
| 2.1 | Acetone | 80 |
| 2.2 | 2-Butanone | 77 |
| 2.3 | Ethyl acetate | 67 |
| 2.4 | Acetonitrile | 64 |
| 2.5 | Dichloromethane | 61 |

### Example 2.1: Oxidation of (R)-Solketal to (S)-glyceraldehyde acetonide in acetone

In a 100 ml 4-necked round-bottomed flask, 5.46 g (40.0 mmole) (R)-Solketal is dissolved in 25.9 g acetone. 3.95 g (48.2 mmole) NaOAc and 3.72 g (16.0 mmole) TCCA are subsequently added and the resulting suspension is stirred at 25 °C.

A solution of 15.7 mg (0.10 mmole) TEMPO in 11.3 g acetone is added over 8 minutes, allowing the reaction mixture to warm up to 59 °C. The reaction mixture is stirred during another 30 minutes and the solids are filtered off and washed with 25 g acetone. GC analysis of the filtrate demonstrates that 4.2 g (32.2 mmole, 80% yield based on (R)-Solketal) (S)-glyceraldehyde acetonide (e.e. = 99.5%) has been obtained.

### Example 2.2: Oxidation of (R,S)-Solketal to (R,S)-glyceraldehyde acetonide in 2-butanone

In a 100 ml 4-necked round-bottomed flask, 4.09 g (30.0 mmole) (R,S)-Solketal is dissolved in 18.9 g 2-butanone. 2.96 g (36.1 mmole) NaOAc and 2.79 g (12.0 mmole) TCCA are subsequently added and the resulting suspension is stirred at 25 °C. A solution of 11.8 mg (0.075 mmole) TEMPO in 8.5 g 2-butanone is added over 9 minutes, allowing the reaction mixture to warm up to 74 °C. The reaction mixture is stirred during another 30 minutes and the solids are filtered off and washed with 20 g 2-butanone. GC analysis of the filtrate demonstrates that 3.0 g (23.2 mmole, 77% yield based on (R,S)-Solketal) (R,S)-glyceraldehyde acetonide has been obtained.

### Example 2.3: Oxidation of (R,S)-Solketal to (R,S)-glyceraldehyde acetonide in ethyl acetate

A solution of 4.09 g (30.0 mmole) (R,S)-Solketal in 19.0 g ethyl acetate is oxidized in ethyl acetate according to the procedure of example 2.2 except that the TEMPO solution is added over 10 minutes, allowing the reaction mixture to warm up to 77 °C and that the solids are washed with ethyl acetate. GC analysis of the filtrate demonstrates that 2.6 g (20.2 mmole, 67% yield based on (R,S)-Solketal) (R,S)-glyceraldehyde acetonide has been obtained.

### Example 2.4: Oxidation of (R,S)-Solketal to (R,S)-glyceraldehyde acetonide in acetonitrile

A solution of 4.09 g (30.0 mmole) (R,S)-Solketal in 19.2 g acetonitrile is oxidized in acetonitrile according to the procedure of example 2.2 except that the TEMPO solution is added over 6 minutes, allowing the reaction mixture to warm up to 60 °C and that the solids are washed with acetonitrile. GC analysis of the filtrate demonstrates that 2.5 g (19.1 mmole, 64% yield based on (R,S)-Solketal) (R,S)-glyceraldehyde acetonide has been obtained.

### Example 2.5: Oxidation of (R,S)-Solketal to (R,S)-glyceraldehyde acetonide in dichloromethane

A solution of 4.09 g (30.0 mmole) (R,S)-Solketal in 28.0 g dichloromethane is oxidized in dichloromethane according to the procedure of example 2.2 except that the TEMPO solution is added over 15 minutes, allowing the reaction mixture to warm up to 42 °C and that the solids are washed with 30 g dichloromethane. GC analysis of the filtrate demonstrates that 2.4 g (18.4 mmole, 61 % yield based on (R,S)-Solketal) (R,S)-glyceraldehyde acetonide has been obtained.

### Influence of the amount of TEMPO

**Table 2: Influence of the amount of TEMPO using 120 mole% NaOAc and 40 mole% TCCA based on Solketal and using acetone as the solvent.**

| Example | TEMPO | Yield (%) of glyceraldehyde acetonide (based on Solketal) |
|---|---|---|
| | (mole% based on Solketal) | |
| 2.6 | 1.0 | 74 |
| 2.7 | 0.33 | 77 |
| 2.1 | 0.25 | 80 |
| 2.8 | 0.15 | 74 |
| 2.9 | 0.081 | 70 |
| 2.10 | 0.023 | 60 |

### Example 2.6: Oxidation of (R,S)-Solketal to (R,S)-glyceraldehyde acetonide with 1.0 mole% TEMPO

In a 100 ml 4-necked round-bottomed flask, 4.09 g (30.0 mmole) (R,S)-Solketal is dissolved in 18.0 g acetone. 2.96 g (36.1 mmole) NaOAc and 2.79 g (12.0 mmole) TCCA are subsequently added and the resulting suspension is stirred at 25 °C. A solution of 48.0 mg (0.30 mmole) TEMPO in 12.2 g acetone is added over 7 minutes, allowing the reaction mixture to warm up to 59 °C. The reaction mixture is stirred during another 30 minutes and the solids are filtered off and washed with 20 g acetone. GC analysis of the filtrate demonstrates that 2.9 g (22.1 mmole, 74% yield based on (R,S)-Solketal) (R,S)-glyceraldehyde acetonide has been obtained.

### Example 2.7: Oxidation of (R,S)-Solketal to (R,S)-glyceraldehyde acetonide with 0.33 mole% TEMPO

A solution of 4.10 g (30.1 mmole) (R,S)-Solketal in 19.3 g acetone is oxidized according to the procedure of example 2.6, except that a solution of 15.8 mg (0.10 mmole) TEMPO in 9.3 g acetone is added over 12 minutes, allowing the reaction mixture to warm up to 60 °C. GC analysis of the filtrate demonstrates that 3.0 g (23.1 mmole, 77% yield based on (R,S)-Solketal) (R,S)-glyceraldehyde acetonide has been obtained.

### Example 2.8: Oxidation of (R,S)-Solketal to (R,S)-glyceraldehyde acetonide with 0.15 mole% TEMPO

A solution of 5.45 g (40.0 mmole) (R,S)-Solketal in 25.6 g acetone is oxidized according to the procedure of example 2.1, except that a solution of 9.4 mg (0.059 mmole) TEMPO in 9.3 g acetone is added over 11 minutes, allowing the reaction mixture to warm up to 59 °C. GC analysis of the filtrate demonstrates that 3.8 g (29.6 mmole, 74% yield based on (R,S)-Solketal) (R,S)-glyceraldehyde acetonide has been obtained.

### Example 2.9: Oxidation of (R,S)-Solketal to (R,S)-glyceraldehyde acetonide with 0.081 mole% TEMPO

In a 100 ml 4-necked round-bottomed flask, 6.14 g (45.0 mmole) (R,S)-Solketal is dissolved in 30.2 g acetone. 4.44 g (54.23 mmole) NaOAc and 4.19 g (18.0 mmole) TCCA are subsequently added and the resulting suspension is stirred at 25 °C. A solution of 5.8 mg (0.036 mmole) TEMPO in 12.3 g acetone is added over 10 minutes, allowing the reaction mixture to warm up to 59 °C. The reaction mixture is stirred during another 30 minutes and the solids are filtered off and washed with 28 g acetone. GC analysis of the filtrate demonstrates that 4.1 g (31.5 mmole, 70% yield based on (R,S)-Solketal) (R,S)-glyceraldehyde acetonide has been obtained.

### Example 2.10: Oxidation of (R,S)-Solketal to (R,S)-glyceraldehyde acetonide with 0.023 mole% TEMPO

A solution of 4.10 g (30.1 mmole) (R,S)-Solketal in 19.7 g acetone is oxidized according to the procedure of example 2.6, except that a solution of 1.1 mg (0.0068 mmole) TEMPO in 8.6 g acetone is added over 10 minutes, allowing the reaction mixture to warm up to 46 °C. GC analysis of the filtrate demonstrates that 2.3 g (18.0 mmole, 60% yield based on (R,S)-Solketal) (R,S)-glyceraldehyde acetonide has been obtained.

### Influence of the amount of TCCA

**Table 3: Influence of the amount of TCCA using 0.25 mole% TEMPO based on Solketal and 300 mole% NaOAc based on TCCA and using acetone as the solvent.**

| Example | TCCA | Yield (%) of glyceraldehyde acetonide |
|---|---|---|
| | (mole% based on Solketal) | (based on Solketal) |
| 2.11 | 33 | 73 |
| 2.1 | 40 | 80 |
| 2.12 | 50 | 77 |
| 2.13 | 100 | 67 |

### Example 2.11: Oxidation of (R,S)-Solketal to (R,S)-glyceraldehyde acetonide with 33 mole% TCCA

In a 100 ml 4-necked round-bottomed flask, 4.10 g (30.1 mmole) (R,S)-Solketal is dissolved in 19.2 g acetone. 2.51 g (30.3 mmole) NaOAc and 2.35 g (10.0 mmole) TCCA (containing 30.0 mmole active chlorine) are subsequently added and the resulting suspension is stirred at 25 °C. A solution of 12.3 mg (0.077 mmole) TEMPO in 8.6 g acetone is added over 8 minutes, allowing the reaction mixture to warm up to 59 °C. After the TEMPO addition is complete, the reaction mixture is cooled to 25 °C over 5 minutes. The solids are filtered off and washed with 25 g acetone. GC analysis of the filtrate demonstrates that 2.9 g (21.9 mmole, 73% yield based on (R,S)-Solketal) (R,S)-glyceraldehyde acetonide has been obtained.

### Example 2.12: Oxidation of (R,S)-Solketal to (R,S)-glyceraldehyde acetonide with 50 mole% TCCA

A solution of 4.09 g (30.0 mmole) (R,S)-Solketal in 22.2 g acetone is oxidized according to the procedure of example 2.11, except that 3.77 g (45.45 mmole) NaOAc and 3.52 g (15.0 mmole) TCCA (containing 45.0 mmole active chlorine) are used and that the TEMPO solution is added over 12 minutes, allowing the reaction mixture to warm up to 59 °C. GC analysis of the filtrate demonstrates that 3.0 g (23.0 mmole, 77% yield based on (R,S)-Solketal) (R,S)-glyceraldehyde acetonide has been obtained.

### Example 2.13: Oxidation of (R,S)-Solketal to (R,S)-glyceraldehyde acetonide with 100 mole% TCCA

In a 100 mL 4-necked round-bottomed flask, 4.44 g (32.6 mmole) (R,S)-Solketal is dissolved in 25.3 g acetone. 8.16 g (98.5 mmole) NaOAc and 7.65 g (32.6 mmole) TCCA (containing 97.8 mmole active chlorine) are subsequently added and the resulting suspension is stirred at 25 °C. A solution of 13.0 mg (0.082 mmole) TEMPO in 9.7 g acetone is added over 9 minutes, allowing the reaction mixture to warm up to 59 °C. GC analysis of the filtrate demonstrates that 2.9 g (21.9 mmole, 67% yield based on (R,S)-Solketal) (R,S)-glyceraldehyde acetonide has been obtained.

### Influence of the amount of NaOAc relative to TCCA

**Table 4: Influence of the amount of NaOAc based on TCCA using 40 mole% TCCA and 0.25 mole% TEMPO based on Solketal and using acetone as the solvent.**

| Example | NaOAc | Yield (%) of glyceraldehyde acetonide (based on Solketal) |
|---|---|---|
| | (mole% based on Solketal) | |
| 2.14 | 80 | 37 |
| 2.15 | 108 | 73 |
| 2.1 | 120 | 80 |

### Example 2.14: Oxidation of (R,S)-Solketal to (R,S)-glyceraldehyde acetonide with 80 mole% NaOAc

In a 100 ml 4-necked round-bottomed flask, 4.09 g (30.0 mmole) (R,S)-Solketal is dissolved in 19.3 g acetone. 2.00 g (24.1 mmole) NaOAc and 2.83 g (12.0 mmole) TCCA are subsequently added and the resulting suspension is stirred at 25 °C. A solution of 12.0 mg (0.075 mmole) TEMPO in 8.9 g acetone is added over 9 minutes, allowing the reaction mixture to warm up to 57 °C. After the TEMPO addition is complete, the reaction mixture is stirred during another 30 minutes. The solids are filtered off and washed with 25 g acetone. GC analysis of the filtrate demonstrates that 1.4 g (11.1 mmole, 37% yield based on (R,S)-Solketal) (R,S)-glyceraldehyde acetonide has been obtained.

### Example 2.15: Oxidation of (R,S)-Solketal to (R,S)-glyceraldehyde acetonide with 108 mole% NaOAc

A solution of 4.09 g (30.0 mmole) (R,S)-Solketal in 19.5 g acetone is oxidized according to the procedure of example 2.14, except that 2.66 g (32.1 mmole) NaOAc is used. After the addition of the TEMPO solution the temperature of the reaction mixture rose to 59 °C. GC analysis of the filtrate demonstrates that 2.8 g (21.7 mmole, 73% yield based on (R,S)-Solketal) (R,S)-glyceraldehyde acetonide has been obtained.

### Influence of the type of base

**Table 5: Influence of the type of base using 40 mole% TCCA and 120 mole% base based on Solketal**

| Example | Base | Solvent | Yield (%) of glyceraldehyde acetonide |
|---|---|---|---|
| | | | (based on Solketal) |
| 2.1 | NaOAc | Acetone | 80 |
| 2.5 | NaOAc | Dichloromethane | 61 |
| 2.16 | NaHCO₃ | Dichloromethane | 44 |

### Example 2.16: Oxidation of (R)-Solketal to (S)-glyceraldehyde acetonide with NaHCO₃ in dichloromethane

In a 100 ml 4-necked round-bottomed flask, 4.19 g (30.7 mmole) (R)-Solketal is dissolved in 24.8 g dichloromethane. 3.07 g (36.2 mmole) NaHCO₃ and 2.84 g (12.1 mmole) TCCA are subsequently added and the resulting suspension is cooled to 3 °C. A solution of 24.1 mg (0.151 mmole) TEMPO in 10.6 g dichloromethane is added over 15 minutes at 3-8 °C. The reaction mixture is stirred during 60 minutes at 5 °C and the solids are filtered off and washed with 25 g dichloromethane. GC analysis of the filtrate demonstrates that 1.8 g (13.6 mmole, 44% yield based on (R)-Solketal) (S)-glyceraldehyde acetonide (e.e. = 99.6%) has been obtained.

### Influence of the addition time of the TEMPO solution

**Table 6: Influence of the addition time of the TEMPO solution using 120 mole% NaOAc, 40 mole% TCCA and 0.25 mole% TEMPO based on Solketal and using acetone as the solvent**

| Example | Dosing time | Yield (%) of glyceraldehyde acetonide |
|---|---|---|
| | (min.) | (based on Solketal) |
| 2.17 | 1.4 | 78 |
| 2.1 | 8 | 80 |

### Example 2.17: Oxidation of (R,S)-Solketal to (R,S)-glyceraldehyde acetonide with fast addition of TEMPO

In a 100 ml 4-necked round-bottomed flask, 4.09 g (30.0 mmole) (R,S)-Solketal is dissolved in 20.2 g acetone. 2.99 g (36.1 mmole) NaOAc and 2.82 g (12.0 mmole) TCCA are subsequently added and the resulting suspension is stirred at 25 °C. A solution of 12.0 mg (0.075 mmole) TEMPO in 6.4 g acetone is added over 85 seconds, allowing the reaction mixture to warm up to 59 °C. The reaction mixture is cooled to 22 °C and the solids are filtered off and washed with 25 g acetone. GC analysis of the filtrate demonstrates that 3.0 g (23.4 mmole, 78% yield based on (R,S)-Solketal) (R,S)-glyceraldehyde acetonide has been obtained.

### Influence of temperature

**Table 7: Influence of the reaction temperature using 120 mole% NaOAc, 40 mole% TCCA and 0.25 mole% TEMPO based on Solketal and using acetone as the solvent**

| Example | Temperature | Yield (%) of glyceraldehyde acetonide |
|---|---|---|
| | | (based on Solketal) |
| 2.18 | 6-15 °C | 59 |
| 2.1 | 25-59 °C | 80 |

### Example 2.18: Oxidation of (R)-Solketal to (S)-glyceraldehyde acetonide at low temperature

In a 100 ml 4-necked round-bottomed flask, 4.10 g (30.1 mmole) (R)-Solketal is dissolved in 24.4 g acetone. 2.99 g (36.1 mmole) NaOAc is added and the suspension is cooled to 1 °C. 2.83 g (12.0 mmole) TCCA is added, while the temperature is kept below 4 °C. The resulting suspension is further cooled to 0 °C and subsequently, a solution of 12.1 mg (0.076 mmole) TEMPO in 7.4 g acetone is added over 9 minutes. The reaction mixture is allowed to reach a maximum temperature of 24 °C, but during at least 90 % of the addition time of the TEMPO solution the temperature was between 15 and 6 °C. After complete addition of the TEMPO solution, the reaction mixture is stirred during 60 minutes between 0 and 6 °C and subsequently warmed up to 20 °C during 60 minutes. The solids are filtered off and washed with 25 g acetone. GC analysis of the filtrate demonstrates that 2.3 g (17.8 mmole, 59% yield based on (R)-Solketal) (S)-glyceraldehyde acetonide (e.e. = 99.6%) has been obtained.

### Influence of addition order

**Table 8:Influence of the addition order using 120 mole% NaOAc, 40 mole% TCCA and 0.25 mole% TEMPO based on Solketal and using acetone as the solvent**

| Example | Dosed reagent | Yield (%) of glyceraldehyde acetonide (based on Solketal) |
|---|---|---|
| 2.19 | TCCA | 67 |
| 2.1 | TEMPO | 80 |

### Example 2.19: Oxidation of (R,S)-Solketal to (R,S)-glyceraldehyde acetonide using addition of TCCA instead of TEMPO

In a 100 ml 4-necked round-bottomed flask, 4.21 g (30.9 mmole) (R,S)-Solketal is dissolved in 16.6 g acetone. 3.09 g (37.3 mmole) NaOAc and 12.3 mg (0.077 mmole) TEMPO are subsequently added and the resulting suspension is stirred at 25 °C. A solution of 2.90 g (12.4 mmole) TCCA acid in 22.6 g acetone is added over 15 minutes, allowing the reaction mixture to warm up to 47 °C. The reaction mixture is stirred during another 60 minutes and the solids are filtered off and washed with 25 g acetone. GC analysis of the filtrate demonstrates that 2.7 g (20.7 mmole, 67% yield based on (R,S)-Solketal) (R,S)-glyceraldehyde acetonide has been obtained.

### Influence of the type of oxidant

**Table 9: Influence of the type of oxidant using 0.25 mole% TEMPO based on Solketal and using acetone as the solvent and NaOAc as the base**

| Example | Oxidizing agent | Amount | Active chlorine | Yield (%) of glyceraldehyde acetonide* |
|---|---|---|---|---|
| | | (mole%*) | (mole%*) | |
| 2.11 | TCCA | 33 | 100 | 73 |
| 2.1 | TCCA | 40 | 120 | 80 |
| 2.12 | TCCA | 50 | 150 | 77 |
| 2.13 | TCCA | 100 | 300 | 67 |
| 2.20 | DCDMH | 90 | 120 | 72 |
| 2.21 | DCDMH | 120 | 160 | 65 |

| | | | | |
|---|---|---|---|---|
| * Based on Solketal | | | | |

### Example 2.20: Oxidation of (R)-Solketal to (S)-glyceraldehyde acetonide with 90 mole% DCDMH

In a 100 ml 4-necked round-bottomed flask, 4.11 g (30.1 mmole) (R)-Solketal is dissolved in 30.1 g acetone. 6.73 g (81.2 mmole) NaOAc and 5.33 g (27.0 mmole) DCDMH (containing 36.0 mmole active chlorine) are subsequently added and the resulting suspension is stirred at 18 °C. A solution of 12.2 mg (0.077 mmole) TEMPO in 8.7 g acetone is added over 9 minutes, allowing the reaction mixture to warm up to 51 °C. The reaction mixture is stirred during another 30 minutes and the solids are filtered off and washed with 25 g acetone. GC analysis of the filtrate demonstrates that 2.8 g (21.6 mmole, 72% yield based on (R)-Solketal) (S)-glyceraldehyde acetonide (e.e. = 99.6%) has been obtained.

### Example 2.21: Oxidation of (R)-Solketal to (S)-glyceraldehyde acetonide with 120 mole% DCDMH

A solution of 4.10 g (30.0 mmole) (R)-Solketal in 30.5 g acetone was oxidized according to the procedure of example 2.20, except that 5.98 g (72.2 mmole) NaOAc and 7.10 g (36.0 mmole) DCDMH (containing 48.0 mmole active chlorine) were used and that the TEMPO solution is added over 10 minutes, allowing the reaction mixture to warm up to 57 °C. GC analysis of the filtrate demonstrates that 2.5 g (19.4 mmole, 65% yield based on (R)-Solketal) (S)-glyceraldehyde acetonide (e.e. = 99.5%) has been obtained.

### Influence of the reaction conditions on the e.e.

**Table 10: Influence of the reaction conditions on the e.e.**

| Ex. | Solvent | Oxidizing agent | Active chlorine (mole%*) | TEMPO (mole%*) | T (°C) | Yield (%) of glyceraldehyde acetonide* | E.e. (%) |
|---|---|---|---|---|---|---|---|
| 2.1 | Acetone | TCCA | 120 | 0.25 | 25-59 | 80 | 99.5 |
| 2.1 6 | Dichloromethane | TCCA | 120 | 0.50 | 3-8 | 44 | 99.6 |
| 2.1 8 | Acetone | TCCA | 120 | 0.25 | 0-24 | 59 | 99.6 |
| 2.2 0 | Acetone | DCDMH | 120 | 0.25 | 18-51 | 72 | 99.6 |
| 2.2 1 | Acetone | DCDMH | 160 | 0.25 | 18-57 | 65 | 99.5 |
| 2.2 2 | Acetone | TCCA | 180 | 0.50 | 25-58 | 71 | 99.6 |

| | | | | | | | |
|---|---|---|---|---|---|---|---|
| * Based on Solketal | | | | | | | |

As can be seen from Table 10, starting from enantiomerically enriched solketal with an enantiomeric excess (e.e.) of 99.6, enantiomerically enriched glyceraldehyde acetonide with similar e.e. can be prepared.

### Example 2.22: Oxidation of (R)-Solketal to (S)-glyceraldehyde acetonide with more TEMPO and TCCA

In a 100 ml 4-necked round-bottomed flask, 4.14 g (30.3 mmole) (R)-Solketal is dissolved in 19.1 g acetone. 4.49 g (54.2 mmole) NaOAc and 4.25 g (18.1 mmole) TCCA are subsequently added and the resulting suspension is stirred at 25 °C. A solution of 24.3 mg (0.15 mmole) TEMPO in 9.0 g acetone is added over 12 minutes, allowing the reaction mixture to warm up to 58 °C. The reaction mixture is stirred during another 105 minutes and the solids are filtered off and washed with 25 g acetone. GC analysis of the filtrate demonstrates that 2.8 g (21.5 mmole, 71 % yield based on (R)-Solketal) (S)-glyceraldehyde acetonide (e.e. = 99.6%) has been obtained.

### Example 2.23: Preparation of (R)-ethyl-((4,5)-O-isopropylidene-(4,5)-dihydroxy)-2-(E,Z)-pentenoate (comparative)

In a 250 ml 4-necked round-bottomed flask, 32.0 g (231.5 mmole) K₂CO₃ is dissolved in 120 ml water. The pH of this solution is adjusted to 11.5 by adding aq. 4 M HCl. The resulting solution is cooled to 3 °C and 8.0 g (34.6 mmole) TEPA is added. While maintaining the temperature of the reaction mixture below 5 °C, 73.5 g of a solution containing 4.2 g (32.0 mmole) (S)-glyceraldehyde acetonide in acetone (as obtained by the procedure in example 2.1) is added over 70 minutes. The pH of the reaction mixture is meanwhile kept in the range 11.0 - 11.5 by adding small portions of K₂CO₃. The resulting mixture is stirred during 4 h between 0 and 5° C and subsequently during 16 h between 5 and 15 °C. GC analysis of the organic phase demonstrates that 5.8 g (28.8 mmole, 90% yield based on (S)-glyceraldehyde acetonide) (R)-ethyl-((4,5)-O-isopropylidene-(4,5)-dihydroxy)-2-(E,Z)-pentenoate (e.e. = 97.8%, E/Z = 95/5) has been obtained.

In conclusion, as is shown by example 2, with the process of the invention glyceraldehyde acetonide can be prepared with a high yield (higher than 30%) and with a high e.e.

## Claims

1. Process for the preparation of glyceraldehyde acetonide by oxidation of 2,2-dimethyl-1,3-dioxolane-4-methanol by an oxidizing agent, **characterized in that** 2,2-dimethyl-1,3-dioxolane-4-methanol is oxidized by an organic N-chloro compound in the presence of an inert base and TEMPO or a TEMPO-derivative of formula 1 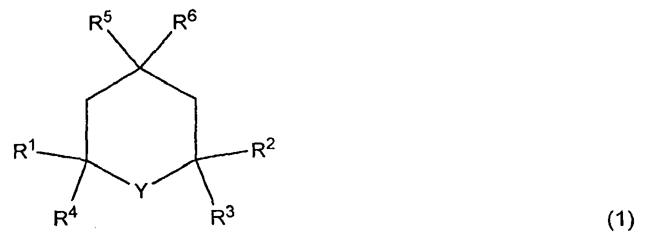 wherein R¹, R², R³ and R⁴ each independently stand for an alkyl group with 1 to 6 C-atoms and wherein R⁵ and R⁶ either both stand for H or an alkoxy group with 1 to 6 C-atoms or one stands for H and the other stands for an alkoxy group with 1 to 6 C-atoms, an alkylcarbonyloxy group with 1 to 6 C-atoms, an arylcarbonyloxy group with the carbonyloxy group having 1 to 6 C-atoms or an alkylcarbonylamino group with 1 to 6 C-atoms; or wherein R⁵ and R⁶ together stand for ketal groups of formula a-c 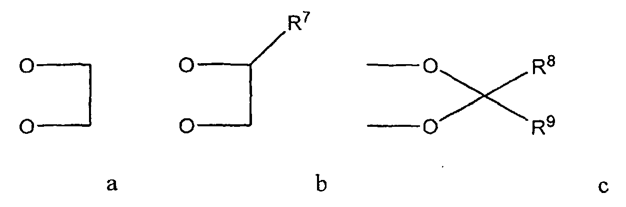 wherein R⁷ stands for an alkyl group with 1 to 6 C-atoms and R⁸ and R⁹ each independently stand for H or an alkyl group with 1 to 6 C-atoms and wherein Y stands for a group of general formula d-f 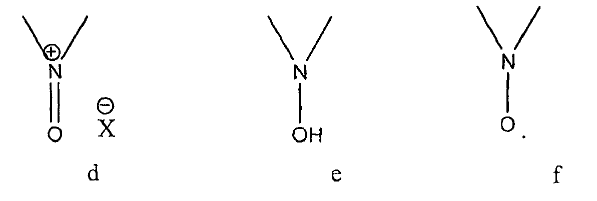 wherein X- stands for an anion.

2. Process according to claim 1, **characterized in that** enantiomerically enriched glyceraldehyde acetonide is prepared by oxidation of the corresponding enantiomerically enriched 2,2-dimethyl-1,3-dioxolane-4-methanol.

3. Process according to claim 1 or claim 2, **characterized in that** the organic N-chloro compound is trichloroisocyanuric acid or dichlorodimethylhydantoin.

4. Process according to any one of claims 1-3, **characterized in that** 2,2-dimethyl-1,3-dioxolane-4-methanol is oxidized in the presence of TEMPO.

5. Process according to any one of claims 1-4, **characterized in that** the inert base has a conjugated acid with a pKₐ > 2.

6. Process according to any one of claims 1-5, **characterized in that** the amount of inert base is at least 0.8 molar equivalent based on the theoretically maximal molar amount of HCl that can be formed in the reaction.

7. Process according to any one of claims 1-6, **characterized in that** the inert base is sodium acetate or sodium bicarbonate.

8. Process according to any one of claims 1-7, **characterized in that** the process is performed at a temperature between 15 and 80°C.

9. Process according to any one of claims 1-8, **characterized in that** the TEMPO or a TEMPO-derivative of formula 1, wherein R¹-R⁶ are as defined above, is added to a mixture of 2,2-dimethyl-1,3-dioxolane-4-methanol, the organic N-chloro compound and the inert base in a solvent.

10. Process according to any one of claims 1-9, **characterized in that** the amount of organic N-chloro compound is such that there is at least 0.5 molar equivalent active chlorine based on the amount of 2,2-dimethyl-1,3-dioxolane-4-methanol.

11. Process according to any one of claims 1-10, **characterized in that** an amount of TEMPO or a TEMPO-derivative of formula 1, wherein R¹-R⁶ are as defined above, of between 0.1 and 1 mole% based on the amount of 2,2-dimethyl-1,3-dioxolane-4-methanol is used.

## Patentansprüche

1. Verfahren zur Herstellung von Glyceraldehydacetonid durch Oxidation von 2,2-Dimethyl-1,3-dioxolan-4-methanol mit einem Oxidationsmittel, **dadurch gekennzeichnet, daß** man 2,2-Dimethyl-1,3-dioxolan-4-methanol mit einer organischen N-Chlorverbindung in Gegenwart einer inerten Base und von TEMPO oder einem TEMPO-Derivat der Formel 1 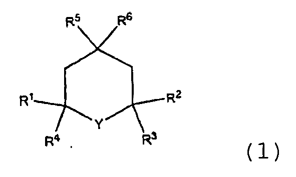 worin R¹, R², R³ und R⁴ jeweils unabhängig voneinander für eine Alkylgruppe mit 1 bis 6 C-Atomen stehen und R⁵ und R⁶ entweder beide für H oder eine Alkoxygruppe mit 1 bis 6 C-Atomen stehen oder eine dieser Gruppen für H steht und die andere für eine Alkoxygruppe mit 1 bis 6 C-Atomen, eine Alkylcarbonyloxygruppe mit 1 bis 6 C-Atomen, eine Arylcarbonyloxygruppe mit 1 bis 6 C-Atomen in der Carbonyloxygruppe oder eine Alkylcarbonylaminogruppe mit 1 bis 6 C-Atomen steht; oder worin R⁵ und R⁶ gemeinsam für Ketalgruppen der Formel a-c 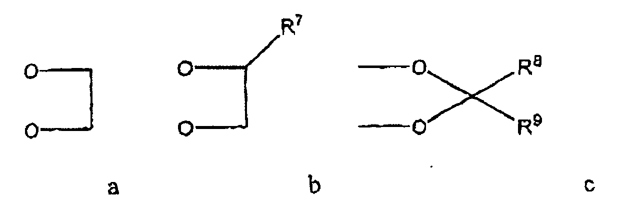 stehen, worin R⁷ für eine Alkylgruppe mit 1 bis 6 C-Atomen steht und R⁸ und R⁹ jeweils unabhängig voneinander für H oder eine Alkylgruppe mit 1 bis 6 C-Atomen stehen, und Y für eine Gruppe der allgemeinen Formel d-f 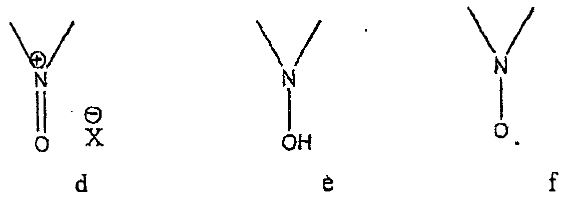 steht, worin X⁻ für ein Anion steht,
oxidiert.

2. Verfahren nach Anspruch 1, **dadurch gekennzeichnet, daß** man durch Oxidation des entsprechenden enantiomerenangereicherten 2,2-Dimethyl-1,3-dioxolan-4-methanols enantiomerenangereichertes Glyceraldehydacetonid herstellt.

3. Verfahren nach Anspruch 1 oder 2, **dadurch gekennzeichnet, daß** es sich bei der organischen N-Chlorverbindung um Trichlorisocyanursäure oder Dichlordimethylhydantoin handelt.

4. Verfahren nach einem der Ansprüche 1-3, **dadurch gekennzeichnet, daß** man 2,2-Dimethyl-1,3-dioxolan-4-methanol in Gegenwart von TEMPO oxidiert.

5. Verfahren nach einem der Ansprüche 1-4, **dadurch gekennzeichnet, daß** die inerte Base eine konjugierte Säure mit einem pKₐ > 2 aufweist.

6. Verfahren nach einem der Ansprüche 1-5, **dadurch gekennzeichnet, daß** die Menge an inerter Base mindestens 0,8 Moläquivalente, bezogen auf die theoretisch maximale molare Menge an HCl, die bei der Reaktion gebildet werden kann, beträgt.

7. Verfahren nach einem der Ansprüche 1-6, **dadurch gekennzeichnet, daß** es sich bei der inerten Base um Natriumacetat oder Natriumhydrogencarbonat handelt.

8. Verfahren nach einem der Ansprüche 1-7, **dadurch gekennzeichnet, daß** man das Verfahren bei einer Temperatur zwischen 15 und 80°C durchführt.

9. Verfahren nach einem der Ansprüche 1-8, **dadurch gekennzeichnet, daß** man das TEMPO oder ein TEMPO-Derivat der Formel 1, worin R¹-R⁶ die oben angegebene Bedeutung besitzen, zu einer Mischung aus 2,2-Dimethyl-1,3-dioxolan-4-methanol, der organischen N-Chlorverbindung und der inerten Base in einem Lösungsmittel gibt.

10. Verfahren nach einem der Ansprüche 1-9, **dadurch gekennzeichnet, daß** die Menge an organischer N-Chlorverbindung so groß ist, daß mindestens 0,5 Moläquivalente aktives Chlor, bezogen auf die Menge an 2,2-Dimethyl-1,3-dioxolan-4-methanol, vorliegen.

11. Verfahren nach einem der Ansprüche 1-10, **dadurch gekennzeichnet, daß** man eine Menge an TEMPO oder TEMPO-Derivat der Formel 1, worin R¹-R⁶ die oben angegebene Bedeutung besitzen, zwischen 0,1 und 1 Mol-%, bezogen auf die Menge an 2,2-Dimethyl-1,3-dioxolan-4-methanol, verwendet.

## Revendications

1. Procédé de préparation de glycéraldéhyde-acétonide par l'oxydation du 2,2-diméthyl-1,3-dioxolane-4-méthanol par un oxydant, **caractérisé en ce que** le 2,2-diméthyl-1,3-dioxolane-4-méthanol est oxydé par un composé N-chloro organique en présence d'une base inerte et de TEMPO ou d'un dérivé de TEMPO de formule 1 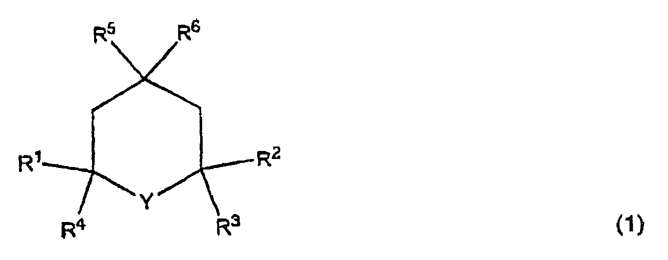 dans laquelle R¹, R², R³ et R⁴ représentent chacun indépendamment un groupe alkyle renfermant de 1 à 6 atomes de carbone et dans laquelle R⁵ et R⁶ soit représentent tous les deux un atome d'hydrogène ou un groupe alcoxy renfermant de 1 à 6 atomes de carbone, soit l'un représente un atome d'hydrogène et l'autre représente un groupe alcoxy renfermant de 1 à 6 atomes de carbone, un groupe alkylcarbonyloxy renfermant de 1 à 6 atomes de carbone, un groupe arylcarbonyloxy avec le groupe carbonyloxy renfermant de 1 à 6 atomes de carbone, ou un groupe alkylcarbonylamino renfermant de 1 à 6 atomes de carbone ; ou dans laquelle R⁵ et R⁶ représentent conjointement des groupes cétal de formules a-c 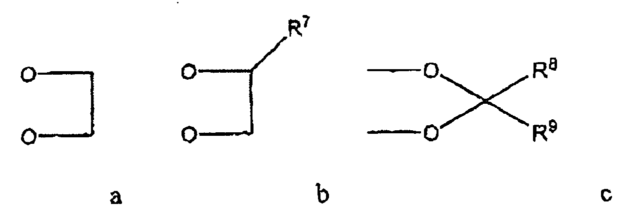 dans lesquelles R⁷ représente un groupe alkyle renfermant de 1 à 6 atomes de carbone, et R⁸ et R⁹ représentent chacun indépendamment un atome d'hydrogène ou un groupe alkyle renfermant de 1 à 6 atomes de carbone, et dans lesquelles Y représente un groupe de formules générales d-f 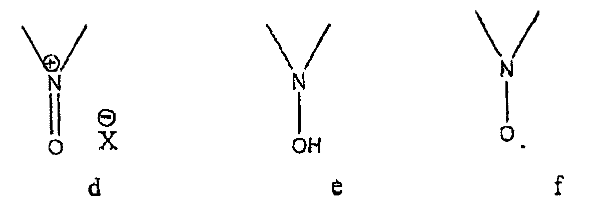 dans lesquelles X⁻ représente un anion.

2. Procédé selon la revendication 1, **caractérisé en ce que** du glycéraldéhyde-acétonide énantiomériquement enrichi est préparé par l'oxydation du 2,2-diméthyl-1,3-dioxolane-4-méthanol énantiomériquement enrichi correspondant.

3. Procédé selon la revendication 1 ou la revendication 2, **caractérisé en ce que** le composé N-chloro organique est l'acide trichloroisocyanurique ou la dichlorodiméthylhydantoïne.

4. Procédé selon l'une quelconque des revendications 1 à 3, **caractérisé en ce que** le 2,2-diméthyl-1,3-dioxolane-4-méthanol est oxydé en présence de TEMPO.

5. Procédé selon l'une quelconque des revendications 1-4, **caractérisé en ce que en ce que** la base inerte présente un acide conjugué présentant un pKₐ > 2.

6. Procédé selon l'une quelconque des revendications 1 à 5, **caractérisé en ce que** la quantité de base inerte est d'au moins 0,8 équivalent molaire, par rapport à la quantité molaire théoriquement maximale de HCl qui peut être formée dans la réaction.

7. Procédé selon l'une quelconque des revendications 1 à 6, **caractérisé en ce que** la base inerte est l'acétate de sodium ou le bicarbonate de sodium.

8. Procédé selon l'une quelconque des revendications 1 à 7, **caractérisé en ce que** le procédé est réalisé à une température comprise entre 15 et 80 °C.

9. Procédé selon l'une quelconque des revendications 1 à 8, **caractérisé en ce que** le TEMPO ou un dérivé de TEMPO de formule 1, dans laquelle R¹ à R⁶ sont tels que définis ci-dessus, est ajouté à un mélange de 2,2-diméthyl-1,3-dioxolane-4-méthanol, du composé N-chloro organique et de la base inerte dans un solvant.

10. Procédé selon l'une quelconque des revendications 1 à 9, **caractérisé en ce que** la quantité de composé N-chloro organique est telle qu'il y ait au moins 0,5 équivalent molaire de chlore actif, par rapport à la quantité de 2,2-diméthyl-1,3-dioxolane-4-méthanol.

11. Procédé selon l'une quelconque des revendications 1-10, **caractérisé en ce qu'**une quantité de TEMPO ou d'un dérivé de TEMPO de formule 1, dans laquelle R¹ à R⁶ sont tels que définis ci-dessus, comprise entre 0,1 et 1 % en moles, par rapport à la quantité de 2,2-diméthyl-1,3-dioxolane-4-méthanol, est utilisée.
